# EUROPEAN PATENT APPLICATION

(11) **EP 4 529 828 A1**
(43) Date of publication of application: **02.04.2025**
(21) Application number: 23199802.2
(22) Date of filing: 26.09.2023
(51) Int. Cl.: A61B 3/13, A61B 90/20, G02B 21/00

(54) **CONTROLLER AND METHOD FOR GENERATING TRAINING DATA, SYSTEMS AND METHODS FOR TRAINING AND APPLICATION OF MACHINE LEARNING ALGORITHMS FOR IMAGES**

(71) Applicant: Leica Instruments (Singapore) Pte Ltd, Singapore 608924 (SG)
(72) Inventor: SORMAZ, Milos, 608924 Singapore (SG)
(74) Representative: Paustian & Partner Patentanwälte mbB

(57) **Abstract**

The present invention essential relates to a controller (150) for a surgical imaging system (100), wherein the surgical imaging system is configured to perform two imaging modes, the two imaging modes comprising a white light imaging mode (120) and a fluorescence imaging mode (126), wherein, in the white light imaging mode, the surgical imaging system is configured to acquire white light images (122) of an object (112), and wherein, in the fluorescence imaging mode (126), the surgical imaging system is configured to acquire fluorescence images (128) of the object (112), wherein the controller (150) is configured to: control the surgical imaging system (100) to perform one of the two imaging modes, and receive the images (122, 128) acquired in said one of the two imaging modes; control the surgical imaging system (100), upon provision of a switching command (124), to perform an imaging mode switch, comprising switching from the one to another of the two imaging modes; control the surgical imaging system (100) to perform said another one of the two imaging modes, and receive the images (122, 128) acquired in said another one of the two imaging modes; generate, when an imaging mode switch is performed, an image pair, the image pair (134) comprising a white light image acquired in the imaging mode before the switch and a fluorescence image acquired in the imaging mode after the switch; and provide the image pair (130) for training of a machine-learning algorithm.

## Description

### Technical Field

The present invention essentially relates to a controller and a method for generating training data for a machine-learning algorithm, based on images, e.g. fluorescence images, from a surgical imaging system, to a system and a method for training such a machine-learning algorithm, to a trained machine-learning algorithm, and to a system and a method for the application of such machine-learning algorithm.

### Background

In surgical microscopy or other kind of surgical imaging, e.g., for tumour surgeries or the like, a surgeon can view the surgical site or the patient by using the surgical imaging system. Tissue or sections thereof to be resected or removed, e.g., tumour tissue, can be supplied with fluorophores or other markers such that relevant sections appear coloured when illuminated with appropriate excitation light. Fluorescence images from such tissue or other objects can be dark.

### Summary

In view of the situation described above, there is a need for improvement in providing fluorescence images. According to embodiments of the invention, a controller and a method for generating training data for a machine-learning algorithm, a system and a method for training a machine-learning algorithm, a trained machine-learning algorithm, and a system and a method for the application of such machine-learning algorithm with the features of the independent claims are proposed. Advantageous further developments form the subject matter of the dependent claims and of the subsequent description.

An embodiment of the invention relates to a controller for a surgical imaging system like a surgical microscope or a surgical endoscope or other kind of imaging system used in surgeries. The surgical imaging system is configured to perform two imaging modes, the two imaging modes comprising a white light imaging mode and a fluorescence imaging mode. In the white light imaging mode, the surgical imaging system is configured to acquire white light images of an object, e.g., tissue of a patient. In the fluorescence imaging mode, the surgical imaging system is configured to acquire fluorescence images of the object.

The controller is configured to control the surgical imaging system to perform one of the two imaging modes, and receive the images acquired in said one of the two imaging modes. This, in particular, means that either the first or the second imaging mode is performed. For example, the surgical imaging system is configured to use only one of the two modes at a time. Further, the controller is configured to control the surgical imaging system, upon provision of a switching command, to perform an imaging mode switch, comprising switching from the one to another of the two imaging modes. This can mean, for example, switching from fluorescence imaging mode to white light imaging mode or vice versa. The switching command can be provided upon user input, e.g., pushing a button or the like. However, the switching command can also be provided automatically, e.g., when a routine or program used with the surgical imaging system requires the change.

The controller is further configured to control the surgical imaging system to perform said another one of the two imaging modes, and receive the images acquired in said another one of the two imaging modes. The controller is further configured to generate, when an imaging mode switch is performed, an image pair; the image pair comprises a first image acquired in the imaging mode before the switch and a second image acquired in the imaging mode after the switch. Such image pair, thus, is a data set comprising the two images. Such image pair comprises a white light image and a fluorescence image, irrespective of which one has been acquired first. The controller is further configured to provide the image pair for training of a machine-learning algorithm. It is noted that these steps can be repeated for multiple image pairs, i.e., upon each switching command, in order to provide sufficient training data.

In an embodiment, the first image is a last frame acquired in the imaging mode before the switch, and the second image is a first frame acquired in the imaging mode after the switch. In this way, the field of view (FOV) is sufficiently equal for both images, irrespective of whether or not care is taken to preserve the FOV for both images. However, other measures can be provided or used to ensure to have the same (or sufficiently the same) FOV for the first image and the second image.

In surgeries, objects like tumour and other harmful tissue can be resected or removed from a patient or a patient's brain, non-harmful tissue or the like. As mentioned before, such (harmful) sections of tissue can be marked by means of fluorophores or other staining material or markers, typically given to the patient. A typical fluorophore for marking harmful tissue like tumour is, but not limited to, 5-ALA (5-aminolevulinic acid). There are also other fluorophores that might be used. During the surgery, the surgical site is then illuminated with excitation light of appropriate wavelength for exciting the fluorophore or marker to emit fluorescence light. This emitted light can be captured or acquired by the surgical imaging system or an imager (image sensor) thereof and, e.g., be displayed on a display. Fluorescence images are used here; the imaging method is also called fluorescence imaging. In this way, the surgeon can easily identify tissue to be resected, or whether tissue to be resected is still present. It is noted that highlighting particular tissue or other objects by means of fluorescence can also have other reason than resection.

While fluorescence signals are often sufficiently bright when viewed via oculars of the surgical imaging system, such that a surgeon can identify relevant regions in the object, it has turned out that fluorescence images, i.e., a digital version of the real fluorescence signals, often are too dark for a surgeon to identify relevant regions in the image. Thus, a surgeon or other users not viewing the surgical site via oculars but via a display, for example, cannot identify relevant regions and not follow the surgery.

Using a machine-learning algorithm, e.g., an artificial neural network, allows increasing brightness of fluorescence images. Such machine-learning algorithm, in turn, needs to be trained before it can be applied, and this requires sufficient and reasonable training data. The way of generating training data as proposed above allows quick and efficient provision of sufficiently good training data. This can, in particular, be automated. It is to be noted that surgical images are of particular kind, e.g., showing a brain like in neurosurgeries. Thus, using any other kind of image pairs, not obtained from surgeries, as training data would not result in sufficiently good results. Since the kind of objects shown in the images is similar irrespective of whether fluorescence or other kind of images are acquired, the proposed way also works for other types of images to be increased in brightness.

According to a further embodiment, the first image is an image acquired in the imaging mode before the switch, within a pre-defined first time period, e.g., 50 ms, before the switch, and the second image is an image acquired in the imaging mode after the switch, within a pre-defined second time period, e.g., also 50 ms, after the switch. In this way, the field of view (FOV) is sufficiently equal for both images, irrespective of whether or not care is taken to preserver the FOV for both images. However, other measures can be provided or used to ensure to have the same (or sufficiently the same) FOV for the first image and the second image, e.g. using one of the last five frames before the switch and one of the first five frames after the switch.

According to a further embodiment, the first image is a last image acquired in the imaging mode before the switch, and wherein the second image is a first image acquired in the imaging mode after the switch. Often, a surgical imaging system acquires images in a series, i.e., in a video stream (a video stream comprises a series of subsequent images). In that a last and first image are chosen, the situation shown therein can be considered to be identical. It is noted that this might require buffering one or more images in order to capture the images before the switch.

Another embodiment of the invention relates to a computer-implemented method generating training data for training of a machine-learning algorithm, using a surgical imaging system. The surgical imaging system is configured to perform two imaging modes, the two imaging modes comprising a white light imaging mode and a fluorescence imaging mode. In the white imaging mode, the surgical imaging system is configured to acquire white light images of an object, and in the fluorescence imaging mode, the surgical imaging system is configured to acquire fluorescence images of the object.

The method comprises controlling the surgical imaging system to perform one of the two imaging modes, and receiving the images acquired in said one of the two imaging modes. Further, the surgical imaging system is controlled, upon provision of a switching command, to perform an imaging mode switch, comprising switching from the one to another of the two imaging modes. The method further comprises controlling the surgical imaging system to perform said another one of the two imaging modes, and receiving the images acquired in said another one of the two imaging modes. The method further comprises generating, when an imaging mode switch is performed, an image pair, the image pair comprising a first image acquired in the imaging mode before the switch and a second image acquired in the imaging mode after the switch. The image pair is provided for training of a machine-learning algorithm.

Another embodiment of the invention relates to a system comprising one or more processors and one or more storage devices, for training of a machine-learning algorithm. The system is configured to receive training data, the training data comprising: multiple image pairs, each image pair comprising a white light and a fluorescence image At least one of the multiple image pairs has been obtained by means of a surgical imaging system when an imaging mode switch has been performed. The imaging mode switch comprises switching between two imaging modes of the surgical imaging system, the two imaging modes comprising a white light imaging mode and a fluorescence imaging mode.

The system is further configured to adjust the machine-learning algorithm (or, weights used therein) based on the training data, such that the machine-learning algorithm increases a brightness of a target fluorescence image. The system is further configured to provide the trained machine learning algorithm for use with said or another surgical imaging system. In this way, an efficient and quick way of training is achieved. Using images that are obtained using a real system and images obtained by it can improve the final outcome.

In an embodiment the machine-learning algorithm is or is based on a RCTNet model.

RCT means Representative Color Transform. Such model can determine different representative colors specialized in input images and estimates transformed colors for the representative colors. It then can determine enhanced colors using these transformed colors based on the similarity between input and representative colors. Further information about this RCTNet model can be found, e.g., in "Hanul Kim et al., Representative Color Transform for Image Enhancement, 2021 IEEE/CVF International Conference on Computer Vision (ICCV), IEEE, 2021."

Another embodiment of the invention relates to a computer-implemented method for training of a machine-learning algorithm. The method comprises receiving training data, the training data comprising: multiple image pairs, each image pair comprising a white light image and a fluorescence image. At least one of the multiple image pairs has been obtained by means of a surgical imaging system when an imaging mode switch has been performed. The imaging mode switch comprises switching between two imaging modes of the surgical imaging system, the two imaging modes comprising a first imaging mode and a second imaging mode. The method further comprises adjusting the machine-learning algorithm based on the training data, such that the machine-learning algorithm increases a brightness of a target fluorescence image. The method further comprises providing the trained machine learning algorithm for use with said or another surgical imaging system.

Another embodiment of the invention relates to a trained machine-learning algorithm for use with a surgical imaging system. The machine-learning algorithm is trained by receiving training data, the training data comprising: multiple image pairs, each image pair comprising a white light image and a fluorescence image. At least one of the multiple image pairs has been obtained by means of a surgical imaging system when an imaging mode switch has been performed. The imaging mode switch comprises switching between two imaging modes of the surgical imaging system, the two imaging modes comprising a first imaging mode and a second imaging mode. The machine-learning algorithm is further trained by adjusting the machine learning algorithm based on the training data, such that the machine-learning algorithm increases a brightness of a target fluorescence image.

Another embodiment of the invention relates to a system comprising one or more processors and one or more storage devices, for increasing a brightness of a fluorescence image. In other words, this system can be used to apply a trained machine-learning algorithm. The system is configured to receive input data, the input data comprising: a fluorescence image obtained by means of a surgical imaging system during a surgery. The system is further configured to increase a brightness of the fluorescence image, by applying the trained machine-learning algorithm mentioned above, in order to obtain a fluorescence image having increased brightness. The system is further configured to provide output data, the output data comprising the fluorescence image having increased brightness. For example, such fluorescence image having increased brightness can be provided on a display to a surgeon or it can be further processed. It is noted that this can be repeated with every fluorescence image received, e.g., from a video stream, resulting in a video stream of fluorescence images having increased brightness.

Another embodiment of the invention relates to a controller for a surgical imaging system, wherein the controller comprises or is configured as the system described above. The controller is configured to control the surgical imaging system to obtain the fluorescence image.

In an embodiment, the controller is configured to increase the brightness of the fluorescence and to provide the output data, i.e., the fluorescence image having increased brightness, in real-time during the surgery. This allows a surgeon or other users not able to view the surgical site via oculars to follow the surgery more conveniently.

In an embodiment, the controller is further configured to record multiple fluorescence images or a stream of multiple fluorescence images obtained by means of the surgical imaging system during the surgery, and to record multiple fluorescence images having increased brightness or a stream of multiple fluorescence images having increased brightness obtained by means applying the machine-learning algorithm. In this way the videos can later be viewed and compared, for example.

Another embodiment of the invention relates to a surgical system, comprising a surgical imaging system with an image sensor and the system as described above or the controller described above. The surgical imaging system is configured to perform a fluorescence imaging mode, wherein, in the fluorescence imaging mode, the surgical imaging system is configured to acquire fluorescence images of the object. The surgical imaging system can also be configured to perform a white light imaging mode, wherein, in the white light imaging mode, the surgical imaging system is configured to acquire white light images of the first type of the object.

It is noted that the surgical imaging system or its controller can be used for both, generating the training data and applying the trained machine-learning algorithm. However, also different but, e.g., similar surgical imaging systems or controllers can be used for the two different aspects. In addition, the training data can, for example, be obtained using a specific surgical imaging system or its controller and the machine-learning algorithm trained based on it can be applied with multiple similar or other surgical imaging systems or its controllers

Another embodiment of the invention relates a computer-implemented method for increasing a brightness of a fluorescence image, i.e., a method for applying the trained machine-learning algorithm. The method comprises receiving input data, input data comprising: a fluorescence image obtained by means of a surgical imaging system during a surgery. The method further comprises increasing a brightness of the fluorescence image, by applying the trained machine-learning algorithm. The method further comprises providing output data, the output data comprising a fluorescence image having increased brightness

With respect to advantages and further embodiments of the methods and controllers, it is referred to the remarks of the systems, which apply here correspondingly.

A further embodiment of the invention relates to a computer program with a program code for performing one of the methods of above, when the computer program is run on a processor.

Further advantages and embodiments of the invention will become apparent from the description and the appended figures.

It should be noted that the previously mentioned features and the features to be further described in the following are usable not only in the respectively indicated combination, but also in further combinations or taken alone, without departing from the scope of the present invention.

### Short description of the Figures

- Fig. 1: schematically shows a system for generating training data for training of a machine-learning algorithm according to an embodiment of the invention;
- Fig. 2: schematically shows a system for training of a machine-learning algorithm according to further embodiment of the invention;
- Fig. 3: schematically shows a system for increasing brightness of an image according to a further embodiment of the invention;
- Fig. 4: schematically shows a way of how to increase brightness of an image according to a further embodiment of the invention;
- Fig. 5: schematically shows a method for generating training data for training of a machine-learning algorithm according to an embodiment of the invention;
- Fig. 6: schematically shows a method for training of a machine-learning algorithm according to a further embodiment of the invention; and
- Fig. 7: schematically shows a method of how to increase brightness according to a further embodiment of the invention.

### Detailed Description

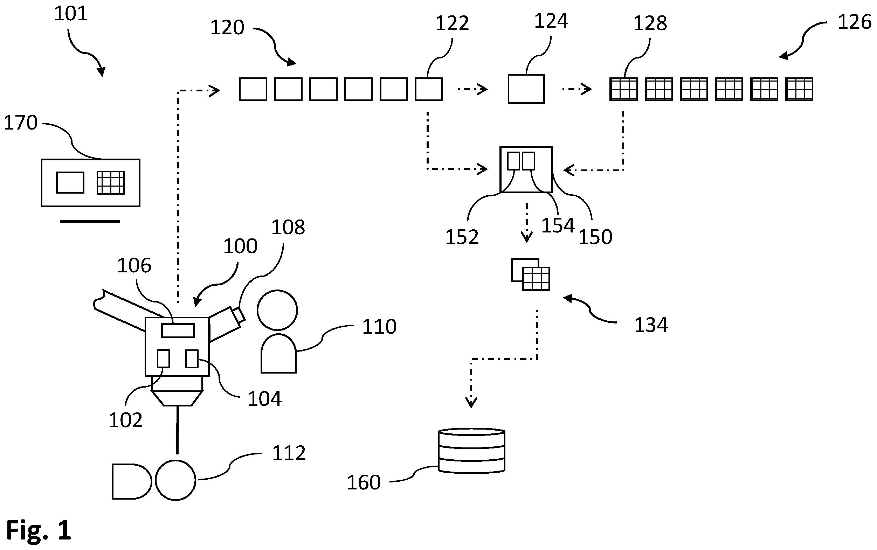

Fig. 1 schematically illustrates a controller 150 for a surgical imaging system 100 for generating training data for training of a machine-learning algorithm according to an embodiment of the invention. The controller 150 comprises one or more processors 152 and one or more storage devices 154. The surgical imaging system 100 and the controller 150 can be part of a surgical system 101.

The surgical microscope 100 is, by means of example, a surgical microscope. The surgical microscope 100 is configured to perform two imaging modes, a white light imaging mode 120 and a fluorescence imaging mode 126. In the white light imaging mode 120, the surgical imaging system 100 is configured to acquire white light images 122 of an object 112, In the fluorescence imaging mode 126, the surgical imaging system 100 is configured to acquire fluorescence images 128 of the object 112.

In an embodiment, said surgical microscope 100 can comprise an illumination optics 102 for visible or white light and an illumination optics 104 for excitation light for exciting fluorophores in tissue of the patient 112. Alternatively, appropriate filters for filtering wavelengths of light required for excitation might be used. An image sensor 106, e.g., a detector or camera, acquires fluorescence light, emanating from the illuminated tissue. Image sensor 106 might also acquire white light. Alternatively, another image sensor can be used for white light.

In this way, the surgical microscope 100 is configured to perform the white light imaging mode 120 in which white light images 122 are acquired, and the fluorescence imaging mode 126, in which fluorescence images 128 are acquired. It is noted that the image sensor 106 might acquire raw data which are then processed to result in the white light images 122 or fluorescence images 128. Such processing of raw data can take place inside the image sensor 106 or another processor included in the surgical microscope 100 or in the controller 150.

In addition, inan embodiment, said surgical microscope 100 can comprise an eyepiece 108 or other appropriate ocular type for directly viewing the object or patient 112 in the currently used imaging mode.

The Controller 150 is configured to control the surgical imaging system 100 to perform one of the two imaging modes, and receive the images acquired in said one of the two imaging modes. This, in particular, means that either the white light imaging mode 120 or the fluorescence imaging mode 126 is performed.

Further, the controller 150 is configured to control the surgical imaging system 100, upon provision of a switching command 124, to perform an imaging mode switch, comprising switching from the one to another of the two imaging modes. In the example shown in Fig. 1, the white light imaging mode 120 is used and then switched to the fluorescence imaging mode 126. The switching command 124 can be received upon a user input, e.g., pressing a button (be it a physical button or software button or any other element). Also, a software routine or other program can trigger such switching command 124.

The controller 150 is further configured to control the surgical imaging system 100 to perform said another one of the two imaging modes, in the example, the fluorescence imaging mode 126, and receive the images acquired in said another one of the two imaging modes, i.e., the fluorescence images 128.

In addition, the controller 150 is configured to generate, when an imaging mode switch is performed, an image pair 134. Such image pair 134 comprises a first image acquired in the imaging mode before the switch and a second image acquired in the imaging mode after the switch. In the example of Fig. 1 the image pair 134 comprises a white light image 122 and a fluorescence image 128. It is noted that if the imaging modes were used vice versa, i.e., switched from the fluorescence imaging mode 126 to the white light imaging mode 120, the image pair 128 would comprise the same kind of images.

In an embodiment, the first image is the last frame acquired in the imaging mode before the switch, and the second image is the first frame acquired in the imaging mode after the switch. In the example of Fig. 1, the first image is the last of the series of white light images 122, and the second image is the first in the series of fluorescence images 128. For example, the images in either mode can be acquired in series to provide a video stream (stream of subsequent images). Then, the last and first frames of these video streams can be chosen for the image pair. Note that this might require buffering of at least one or more image (frames) acquired in the imaging mode before the switch, such that upon the switch, the relevant image is available.

The image pair 134 is then provided for training of a machine-learning algorithm, wherein the machine-learning algorithm is to be trained for use with said surgical imaging system 100 or another surgical imaging system. It is noted that multiple of such image pair can be provided, e.g., upon each switching command, in order to provide sufficient training data. Such multiple image pairs are then also called training data.

In the following, it will be explained in more detail and based on an example how to obtain said image pairs 134, referring to Fig. 1. During a surgery, a surgeon 110 (user) uses the surgical microscope 100 (or another surgical imaging system) in order to view the surgical site, e.g., a patient 112 or a patient's brain.

The controller 150 is configured to control the surgical microscope 100 to perform either the white light imaging mode 120 or the fluorescence imaging mode 126, which is chose to be used by the surgeon 110, for example.

Every time a switch between the two imaging modes is performed, an image pair 134 comprising the white light image 120 and the fluorescence image 128 can be generated and provided as training data or part of it. For example, the image pairs 134 can be stored in a database 160 until required for training. Using the switch between the imaging modes as a trigger and, in particular, choosing the last and first frames, helps ensuring to have the same field of view of both images.

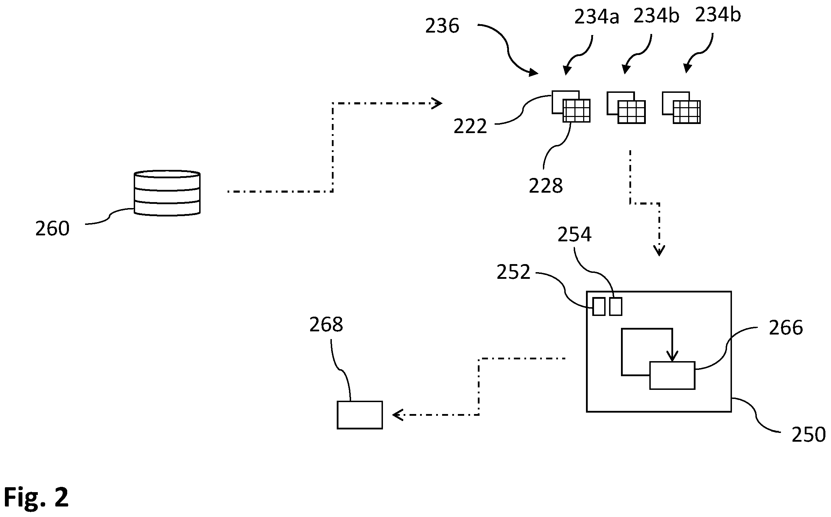

Fig. 2 schematically illustrates a system 250 for training of a machine-learning algorithm according to a further embodiment of the invention. The system 250 comprises one or more processors 252 and one or more storage devices 254. The system 250 can, for example, be a computer or a server, e.g., in the cloud (cloud computing).

The system 250 is configured to receive training data 236, e.g., from a database 260. The training data 236 comprises multiple image pairs. By means of example, three image pairs 234a, 234b, 234c are shown in Fig. 2. A typical number of images pairs used as training data can be, for example, hundreds or thousands of image pairs. Each image pair 234a, 234b, 234c comprises a white light image 220 of an object and a fluorescence image 228 of the object, wherein the images have been obtained by means of a surgical imaging system, upon a switch of the imaging modes, as described with respect to Fig. 1, for example.

Each or some of the image pairs can correspond to the image pairs 134 described with respect to Fig. 1; this, in particular includes the way of how to obtain such image pairs. It is noted that not all image pairs need to be acquired using the same surgical imaging system, rather, two or more different surgical imaging systems can be used.

The system 250 is further configured to adjust the machine-learning algorithm 260 based on the training data 236, such that the machine-learning algorithm 260 increases a brightness of a target fluorescence image. For the training of the machine-learning algorithm, e.g., weights of the machine-learning algorithm (or model or neural network) are adapted. The white light images can be used as ground truth. The system 250 then provides the trained machine learning algorithm 268 for use with the surgical imaging system that has been used for generating the training data, e.g., surgical imaging system 150 of Fig. 1. However, the trained machine learning algorithm 266 can also be provided for use with another similar or even other type of surgical imaging system.

In an embodiment the machine-learning algorithm is or is based on a RCTNet model. RCT means Representative Color Transform.

For training, the fluorescence images 232 of an image pair can be used as input to the machine-learning algorithm, and the white light images of the respective image pairs can be used as a desired output of the machine-learning algorithm. As mentioned, the fluorescence images are darker than the white light images. The machine-learning algorithm shall, thus, increase the brightness in a fluorescence image (or, rather, an image).

In order to obtain this, e.g., weights of the machine-learning algorithm - it may be an artificial neural network - are to be adjusted. For example, such weights might be adjusted iteratively until a version having increased brightness of an input fluorescence image, created by the machine-learning algorithm, corresponds (at least within pre-defined limits; e.g., mean square error can be minimized) to the provided white light image.

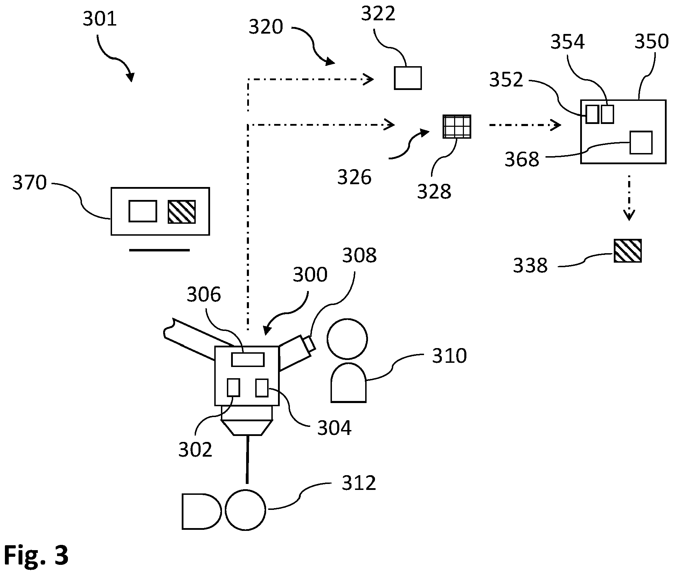

Fig. 3 schematically illustrates a system 350 for increasing a brightness of an image of a second type, e.g., a fluorescence image. This is an application of a (trained) machine-learning algorithm. The system 350 comprises one or more processors 352 and one or more storage devices 354. In an embodiment, system 350 is configured as a controller for a surgical imaging system 300 also shown in Fig. 3. The surgical imaging system 300 and the controller 350 can be part of a surgical system 301.

System 350 is configured to receive input data. The input data comprises a fluorescence image 328, obtained by means of a surgical imaging system 300 during a surgery. The system 350 then increases the brightness of said fluorescence image 328 (the target fluorescence image), by applying the trained machine-learning algorithm 368, in order to obtain a fluorescence image 338 having increased brightness. The trained machine-learning algorithm 368 can correspond to, for example, the trained machine-learning algorithm 268 obtained as described with respect to Fig. 2. The system 350 then provides output data. The output data comprises the fluorescence image 338 having increased brightness. The fluorescence image 338 having increased brightness can then, e.g., be displayed on a display 370 for a surgeon.

In the following, it will be explained in more detail and based on an example how to obtain said fluorescence image 328, and said fluorescence image 338 having increased brightness, referring to Fig. 3

During a surgery, a surgeon 310 (user) uses the surgical microscope 300 (or another surgical imaging system) in order to view the surgical site, e.g., a patient 312 or a patient's brain. Said surgical microscope 300 can comprise an illumination optics 302 for visible or white light and an illumination optics 304 for excitation light for exciting fluorophores in tissue of the patient 312. Alternatively, appropriate filters for filtering wavelengths of light required for excitation might be used. An image sensor 306, e.g., a detector or camera, acquires fluorescence light, emanating from the illuminated tissue. Image sensor 306 might also acquire white light. Alternatively, another image sensor can be used for white light.

In this way, the surgical microscope 300 is configured to perform a white light imaging mode 320 in which white light images 322 are acquired, and a fluorescence imaging mode 326, in which fluorescence images 328 are acquired. It is noted that the image sensor 306 might acquire raw data which are then processed to result in the white light images 322 or fluorescence images 328. Such processing of raw data can take place inside the image sensor 306 or another processor included in the surgical microscope 300 or in the system or controller 350.

It is noted that such white light images 322 or fluorescence images 328 can be produced or acquired during the surgery several times, e.g., within a video stream, depending on the mode used.

Every time or, e.g., only when desired, an fluorescence image 328 is acquired, its brightness is increased, using or applying the trained machine-learning algorithm 368 as explained above, in order to obtain the fluorescence image 338 having increased brightness, which can then be, for example, displayed on the display 370 of the surgical system 301 for assistance of the surgeon and/or other users watching the surgery via the display, while the surgeon uses an eyepiece 308 of the surgical microscope.

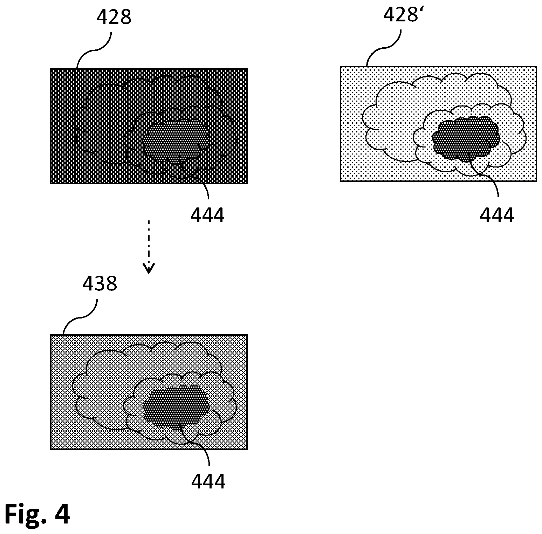

Fig. 4 schematically illustrates a way of how to increase brightness of an image, e.g. a fluorescence image, according to a further embodiment of the invention. The situation shown is basically similar to the one of Fig.3, however, more detailed images are shown.

A fluorescence image 428 can be obtained, for example, by means of a surgical imaging system like described with respect to Fig. 3. By means of example, the fluorescence image 428 shows an object like tissue, e.g., a brain of a patient. A region 444 of the object in the image is illustrated differently, which indicates that this region exhibits the fluorophore generating the actual fluorescence signal. Such fluorescence image 428, however, is often very dark such that the anatomical structures around the region 444 are hardly visible.

For comparison, a view 428' is illustrated which shows the view of, e.g., the surgeon using the eyepiece of the surgical microscope. This real view is brighter than the corresponding image, allowing to see the anatomical structures around the region 444 much better than in the image 428.

Using the trained machine-learning algorithm as explained above, the brightness of the fluorescence image 428 can be increased, resulting in fluorescence image 438 having increased brightness. This does not mean that the fluorescence image 438 is equivalent to the real view 428', however, the anatomical structure surrounding the region 444 are much better visible than in the original fluorescence image 428.

It is noted that described steps can be performed in real-time during the surgery, i.e., for every new fluorescence image obtained, such that the surgeon or, rather, other users, always can be provided with a current view.

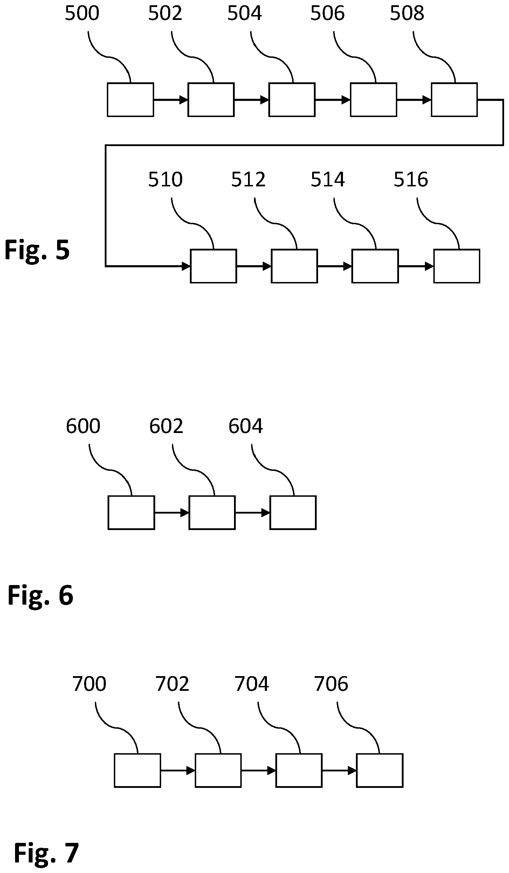

Fig. 5 schematically illustrates a computer-implemented method for generating training data for training of a machine-learning algorithm. A surgical imaging system configured to perform two imaging modes, a first imaging mode and a second imaging mode, is used. For example, the surgical imaging system described with respect to Fig. 1 can be used.

In a step 500, the surgical imaging system is controlled to perform one of the two imaging modes, and, step 502, the images acquired in said one of the two imaging modes can be received and, e.g., buffered or stored. In step 504, switching command is received, and, step 506, the surgical microscope is controlled to perform an imaging mode switch, comprising switching from the one to another of the two imaging modes.

In step 508, the surgical imaging system is controlled to perform said another one of the two imaging modes, and, step 510, the images acquired in said another one of the two imaging modes can be received and, e.g., buffered or stored. In step 512, when an imaging mode switch is performed, an image pair is generated, wherein the image pair comprises a first image acquired in the imaging mode before the switch and a second image acquired in the imaging mode after the switch. In step 514, the image pair is provided for training of a machine-learning algorithm. For example, in step 516, the image pair is then stored in a data base for later use. Steps 500 to 516 can be repeated for every switch image performed when using a surgical imaging system.

Fig. 6 schematically illustrates a computer-implemented method for training of a machine-learning algorithm. In step 600, training data is received. The training data comprises multiple image pairs, each image pair comprising an image of a first type and an image of a second type, wherein the images of the second type are, at least in average, darker than the images of the first type. For example, each image pair can comprise a fluorescence image and a white light image of the object. For at least one of the multiple image pairs, preferably for more or all of them, the images have been obtained by means of a surgical imaging system.

In Step 602, the machine-learning algorithm is adjusted (trained), based on the training data, such that the machine-learning algorithm (later, when applied) increases a brightness of a target image of the second type, e.g., a target fluorescence image. The white light images (or images of the first type) can used as ground truth. This step can comprise, for example, adjusting weights of a neural network. In step 604, the trained machine learning algorithm is provided for use with said or another surgical imaging system.

Fig. 7 schematically illustrates a computer-implemented method for increasing a brightness of an image of a second type (or, rather, just an image), i.e., an application of the machine-learning algorithm. In step 700, input data is received; the input data comprises an image of the second type obtained by means of a surgical microscope during a surgery, e.g., a fluorescence image. In step 702, brightness of the image of the second type or fluorescence image is increased, by applying the trained machine-learning algorithm, in order to obtain an image of the second type (or fluorescence image) having increased brightness. In step 704, output data is provided; the output data comprises the image of the second type having increased brightness.

In an embodiment, in step 706, a display is controlled to show said image of the second type having increased brightness on it.

As used herein the term "and/or" includes any and all combinations of one or more of the associated listed items and may be abbreviated as "f".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Some embodiments relate to a surgical imaging system comprising a surgical system as described in connection with one or more of the Figs. 1 to 7. Alternatively, a surgical imaging system may be part of or connected to a surgical system as described in connection with one or more of the Figs. 1 to 7. Fig. 1 shows a schematic illustration of a surgical system 101 configured to perform a method described herein. The surgical system 101 comprises a surgical imaging system 100 and a computer system or controller 150. The surgical imaging system 100 is configured to take images and is connected to the computer system 150. The computer system 150 is configured to execute at least a part of a method described herein. The computer system 150 may be configured to execute a machine learning algorithm. The computer system 150 and surgical imaging system 100 may be separate entities but can also be integrated together in one common housing. The computer system 150 may be part of a central processing system of the surgical imaging system 100 and/or the computer system 150 may be part of a subcomponent of the surgical imaging system 100, such as a sensor, an actor, a camera or an illumination unit, etc. of the surgical imaging system 100.

The computer system 150 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 150 may comprise any circuit or combination of circuits. In one embodiment, the computer system 150 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system X20 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system X20 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 150 may also include a display device, one or more speakers, and a keyboard and/or controller, which can include a mouse, trackball, touch screen, voice-recognition device, or any other device that permits a system user to input information into and receive information from the computer system 150.

This applies correspondingly to surgical system 301, surgical imaging system 300 and computer system 350.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

Embodiments may be based on using a machine-learning model or machine-learning algorithm. Machine learning may refer to algorithms and statistical models that computer systems may use to perform a specific task without using explicit instructions, instead relying on models and inference. For example, in machine-learning, instead of a rule-based transformation of data, a transformation of data may be used, that is inferred from an analysis of historical and/or training data. For example, the content of images may be analyzed using a machine-learning model or using a machine-learning algorithm. In order for the machine-learning model to analyze the content of an image, the machine-learning model may be trained using training images as input and training content information as output. By training the machine-learning model with a large number of training images and/or training sequences (e.g. words or sentences) and associated training content information (e.g. labels or annotations), the machine-learning model "learns" to recognize the content of the images, so the content of images that are not included in the training data can be recognized using the machine-learning model. The same principle may be used for other kinds of sensor data as well: By training a machine-learning model using training sensor data and a desired output, the machine-learning model "learns" a transformation between the sensor data and the output, which can be used to provide an output based on non-training sensor data provided to the machine-learning model. The provided data (e.g. sensor data, meta data and/or image data) may be preprocessed to obtain a feature vector, which is used as input to the machine-learning model.

Machine-learning models may be trained using training input data. The examples specified above use a training method called "supervised learning". In supervised learning, the machine-learning model is trained using a plurality of training samples, wherein each sample may comprise a plurality of input data values, and a plurality of desired output values, i.e. each training sample is associated with a desired output value. By specifying both training samples and desired output values, the machine-learning model "learns" which output value to provide based on an input sample that is similar to the samples provided during the training. Apart from supervised learning, semi-supervised learning may be used. In semi-supervised learning, some of the training samples lack a corresponding desired output value. Supervised learning may be based on a supervised learning algorithm (e.g. a classification algorithm, a regression algorithm or a similarity learning algorithm. Classification algorithms may be used when the outputs are restricted to a limited set of values (categorical variables), i.e. the input is classified to one of the limited set of values. Regression algorithms may be used when the outputs may have any numerical value (within a range). Similarity learning algorithms may be similar to both classification and regression algorithms but are based on learning from examples using a similarity function that measures how similar or related two objects are. Apart from supervised or semi-supervised learning, unsupervised learning may be used to train the machine-learning model. In unsupervised learning, (only) input data might be supplied and an unsupervised learning algorithm may be used to find structure in the input data (e.g. by grouping or clustering the input data, finding commonalities in the data). Clustering is the assignment of input data comprising a plurality of input values into subsets (clusters) so that input values within the same cluster are similar according to one or more (pre-defined) similarity criteria, while being dissimilar to input values that are included in other clusters.

Reinforcement learning is a third group of machine-learning algorithms. In other words, reinforcement learning may be used to train the machine-learning model. In reinforcement learning, one or more software actors (called "software agents") are trained to take actions in an environment. Based on the taken actions, a reward is calculated. Reinforcement learning is based on training the one or more software agents to choose the actions such, that the cumulative reward is increased, leading to software agents that become better at the task they are given (as evidenced by increasing rewards).

Furthermore, some techniques may be applied to some of the machine-learning algorithms. For example, feature learning may be used. In other words, the machine-learning model may at least partially be trained using feature learning, and/or the machine-learning algorithm may comprise a feature learning component. Feature learning algorithms, which may be called representation learning algorithms, may preserve the information in their input but also transform it in a way that makes it useful, often as a pre-processing step before performing classification or predictions. Feature learning may be based on principal components analysis or cluster analysis, for example.

In some examples, anomaly detection (i.e. outlier detection) may be used, which is aimed at providing an identification of input values that raise suspicions by differing significantly from the majority of input or training data. In other words, the machine-learning model may at least partially be trained using anomaly detection, and/or the machine-learning algorithm may comprise an anomaly detection component.

In some examples, the machine-learning algorithm may use a decision tree as a predictive model. In other words, the machine-learning model may be based on a decision tree. In a decision tree, observations about an item (e.g. a set of input values) may be represented by the branches of the decision tree, and an output value corresponding to the item may be represented by the leaves of the decision tree. Decision trees may support both discrete values and continuous values as output values. If discrete values are used, the decision tree may be denoted a classification tree, if continuous values are used, the decision tree may be denoted a regression tree. Association rules are a further technique that may be used in machine-learning algorithms. In other words, the machine-learning model may be based on one or more association rules. Association rules are created by identifying relationships between variables in large amounts of data. The machine-learning algorithm may identify and/or utilize one or more relational rules that represent the knowledge that is derived from the data. The rules may e.g. be used to store, manipulate or apply the knowledge. Machine-learning algorithms are usually based on a machine-learning model. In other words, the term "machine-learning algorithm" may denote a set of instructions that may be used to create, train or use a machine-learning model. The term "machine-learning model" may denote a data structure and/or set of rules that represents the learned knowledge (e.g. based on the training performed by the machine-learning algorithm). In embodiments, the usage of a machine-learning algorithm may imply the usage of an underlying machine-learning model (or of a plurality of underlying machine-learning models). The usage of a machine-learning model may imply that the machine-learning model and/or the data structure/set of rules that is the machine-learning model is trained by a machine-learning algorithm.

For example, the machine-learning model may be an artificial neural network (ANN). ANNs are systems that are inspired by biological neural networks, such as can be found in a retina or a brain. ANNs comprise a plurality of interconnected nodes and a plurality of connections, so-called edges, between the nodes. There are usually three types of nodes, input nodes that receiving input values, hidden nodes that are (only) connected to other nodes, and output nodes that provide output values. Each node may represent an artificial neuron. Each edge may transmit information, from one node to another. The output of a node may be defined as a (non-linear) function of its inputs (e.g. of the sum of its inputs). The inputs of a node may be used in the function based on a "weight" of the edge or of the node that provides the input. The weight of nodes and/or of edges may be adjusted in the learning process. In other words, the training of an artificial neural network may comprise adjusting the weights of the nodes and/or edges of the artificial neural network, i.e. to achieve a desired output for a given input. Alternatively, the machine-learning model may be a support vector machine, a random forest model or a gradient boosting model. Support vector machines (i.e. support vector networks) are supervised learning models with associated learning algorithms that may be used to analyze data (e.g. in classification or regression analysis). Support vector machines may be trained by providing an input with a plurality of training input values that belong to one of two categories. The support vector machine may be trained to assign a new input value to one of the two categories. Alternatively, the machine-learning model may be a Bayesian network, which is a probabilistic directed acyclic graphical model. A Bayesian network may represent a set of random variables and their conditional dependencies using a directed acyclic graph. Alternatively, the machine-learning model may be based on a genetic algorithm, which is a search algorithm and heuristic technique that mimics the process of natural selection.

### List of Reference Signs

- 100, 300: surgical imaging system
- 101, 301: surgical system
- 102, 104, 302, 304: illumination optics
- 106, 306: imaging sensor
- 108, 308: eyepiece
- 110,310: surgeon
- 112, 312: patient
- 120, 320: first imaging mode
- 122, 222: white light image
- 126, 326: second imaging mode
- 128, 228, 328, 428: fluorescence image
- 134, 234a, 234b, 234c: image pair
- 150: controller
- 152, 252, 352: processor
- 154, 254, 354: storage device
- 160,260: database
- 170,370: display

- 250,350: system
- 266: machine-learning algorithm
- 268, 368: trained machine-learning algorithm

- 338, 438: fluorescence image having increased brightness

- 428': real view
- 444: region

- 500-516, 600-604, 700-706: method steps

## Claims

1. A controller (150) for a surgical imaging system (100), wherein the surgical imaging system is configured to perform two imaging modes, the two imaging modes comprising a white light imaging mode (120) and a fluorescence imaging mode (126),
wherein, in the white light imaging mode, the surgical imaging system is configured to acquire white light images (122) of an object (112), and
wherein, in the fluorescence imaging mode (126), the surgical imaging system is configured to acquire fluorescence images (128) of the object (112),
wherein the controller (150) is configured to:
control the surgical imaging system (100) to perform one of the two imaging modes, and receive the images (122, 128) acquired in said one of the two imaging modes;
control the surgical imaging system (100), upon provision of a switching command (124), to perform an imaging mode switch, comprising switching from the one to another of the two imaging modes;
control the surgical imaging system (100) to perform said another one of the two imaging modes, and receive the images (122, 128) acquired in said another one of the two imaging modes;
generate, when an imaging mode switch is performed, an image pair, the image pair (134) comprising a white light image acquired in the imaging mode before the switch and a fluorescence image acquired in the imaging mode after the switch; and
provide the image pair (130) for training of a machine-learning algorithm.

2. The controller (150) of claim 1, wherein the first image acquired in the imaging mode before the switch , and the second image acquired in the imaging mode after the switch, have been acquired with the same field of view.

3. The controller (150) of claim 1 or 2, wherein the first image is a last frame acquired in the imaging mode before the switch, and wherein the second image is a first frame acquired in the imaging mode after the switch.

4. A computer-implemented method for generating training data for training of a machine-learning algorithm, using a surgical imaging system (100) configured to perform two imaging modes, the two imaging modes comprising a white light imaging mode (120) and a fluorescence imaging mode (126),
wherein, in the white light imaging mode, the surgical imaging system is configured to acquire white light images (122) of an object (112), and
wherein, in the fluorescence imaging mode (126), the surgical imaging system is configured to acquire fluorescence images (128) of the object (112),
the method comprising:
controlling (500) the surgical imaging system (100) to perform one of the two imaging modes, and receiving (502) the images (122, 128) acquired in said one of the two imaging modes;
controlling (506) the surgical microscope (100), upon provision of a switching command (124), to perform an imaging mode switch, comprising switching from the one to another of the two imaging modes;
controlling (508) the surgical imaging system (100) to perform said another one of the two imaging modes, and receiving (510) the images (122, 128) acquired in said another one of the two imaging modes;
generating (512), when an imaging mode switch is performed, an image pair, the image pair (130) comprising a first image acquired in the imaging mode before the switch and a second image acquired in the imaging mode after the switch; and
providing (514) the image pair (130) for training of a machine-learning algorithm.

5. A system (250) comprising one or more processors (252) and one or more storage devices (254), for training of a machine-learning algorithm, wherein the system is configured to:
receive training data (236), the training data comprising: multiple image pairs (234a, 234b, 234c), each image pair comprising a white light image of a first type (220) and a fluorescence image of (228), wherein at least one of the multiple image pairs has been obtained by means of a surgical imaging system when an imaging mode switch has been performed, wherein the imaging mode switch comprises switching between two imaging modes of the surgical imaging system, the two imaging modes comprising a first imaging mode and a second imaging mode;
adjust the machine-learning algorithm (266) based on the training data, such that the machine-learning algorithm increases a brightness of a target fluorescence image; and
provide the trained machine learning algorithm (268) for use with said or another surgical imaging system.

6. The system of claim 5, wherein the machine-learning algorithm is or is based on a RCTNet model.

7. A computer-implemented method for training of a machine-learning algorithm, comprising:
receiving (600) training data, the training data comprising: multiple image pairs, each image pair comprising a white light image and a fluorescence image of a second type, wherein at least one of the multiple image pairs has been obtained by means of a surgical imaging system when an imaging mode switch has been performed, wherein the imaging mode switch comprises switching between two imaging modes of the surgical imaging system, the two imaging modes comprising a first imaging mode and a second imaging mode;
adjusting (602) the machine-learning algorithm based on the training data, such that the machine-learning algorithm increases a brightness of a target image of the second type; and
providing (604) the trained machine learning algorithm for use with said or another surgical imaging system.

8. A trained machine-learning algorithm for use with a surgical imaging system, trained by:
receiving training data, the training data comprising: multiple image pairs, each image pair comprising a white light image and a fluorescence image, wherein at least one of the multiple image pairs has been obtained by means of said or another surgical imaging system when an imaging mode switch has been performed, wherein the imaging mode switch comprises switching between two imaging modes of said another surgical imaging system, the two imaging modes comprising a first imaging mode and a second imaging mode; and
adjusting the machine learning algorithm based on the training data, such that the machine-learning algorithm increases a brightness of a target fluorescence image.

9. A system (350) comprising one or more processors (352) and one or more storage devices (354), for increasing a brightness of a fluorescence image, wherein the system (350) is configured to:
receive input data, the input data comprising: a fluorescence image(328, 428) obtained by means of a surgical imaging system (300) during a surgery,
increase a brightness of the fluorescence image, by applying the trained machine-learning algorithm (368) of claim 8; and
provide output data, the output data comprising: a fluorescence image having increased brightness (338, 438).

10. A controller for a surgical imaging system (300), wherein the controller comprises or is configured as the system (350) of claim 9, wherein the controller is configured to: control the surgical imaging system to obtain the fluorescence image.

11. The controller of claim 10, configured to increase the brightness of the fluorescence image (328) and to provide the output data in real-time during the surgery.

12. The controller of claim 10 or 11, further configured to:
record multiple fluorescence images or a stream of multiple fluorescence images obtained by means of the surgical imaging system during the surgery; and
record multiple fluorescence images having increased brightness or a stream of multiple fluorescence images having increased brightness obtained by means applying the machine-learning algorithm.

13. A surgical system (101, 301), comprising a surgical imaging system (100, 300) with an image sensor and the system (350) of any one of claims 1 to 3 or 9 or the controller (150) of any one of claims 4 or 10 to 12, wherein the surgical imaging system is configured to perform a fluorescence imaging mode, wherein, in the fluorescence imaging mode, the surgical imaging system is configured to acquire fluorescence images of the object.

14. A computer-implemented method for increasing a brightness of a fluorescence image, comprising:
Receiving (700) input data, the input data comprising: a fluorescence image obtained by means of a surgical microscope during a surgery,
Increasing (702) a brightness of the fluorescence image, by applying the trained machine-learning algorithm of claim 8; and
providing (704) output data, the output data comprising: a fluorescence image having increased brightness.

15. A computer program with a program code for performing the method of claim 4, 7 or 14, when the computer program is run on a processor.
